Europäisches Patentamt

European Patent Office

Office européen des brevets

(11) Veröffentlichungsnummer: **0 475 027 A1**

(12) **EUROPÄISCHE PATENTANMELDUNG**

(21) Anmeldenummer: **91112258.8**

(22) Anmeldetag: **22.07.91**

(51) Int. Cl.⁵: **A61N 1/05**

(30) Priorität: **02.08.90 DE 4024597**

(43) Veröffentlichungstag der Anmeldung:
**18.03.92 Patentblatt 92/12**

(84) Benannte Vertragsstaaten:
**DE FR GB IT NL SE**

(71) Anmelder: **SIEMENS AKTIENGESELLSCHAFT**
**Wittelsbacherplatz 2**
**W-8000 München 2(DE)**

(72) Erfinder: **Mund, Konrad, Dr.**
**Langenbrucker Weg 10**
**W-8525 Uttenreuth(DE)**
Erfinder: **Freller, Helmut**
**Steinbergstrasse 34a**
**W-8505 Röthenbach/Pegnitz(DE)**

(54) **Elektroden für implantierbare Defibrillatoren.**

�ហ Bei Elektroden für implantierbare Defibrillatoren in Form eines dünnen Titannetzes, die eine gleichbleibend hohe Doppelschichtkapazität aufweisen und an denen keine Gasentwicklung erfolgt, weist das Titannetz eine poröse Schicht aus einem Carbid, Nitrid oder Carbonitrid oder einem elektronisch leitenden Oxid wenigstens eines der Metalle aus der Gruppe der Elemente der vierten und fünften Nebengruppe des Periodensystems und Wolfram auf.

EP 0 475 027 A1

Die Erfindung betrifft Elektroden für implantierbare Defibrillatoren in Form eines dünnen Titannetzes sowie ein Verfahren zur Herstellung derartiger Elektroden.

Verschiedene Funktionsanomalien des Herzens führen zu einem unregelmäßigen Herzschlag (Arrhythmie) bzw. zu Herzflattern oder -flimmern (Tachykardie). Wenn das Herz eines Patienten flattert oder fibrilliert, dann ist der koordinierte Bewegungsablauf des Herzmuskels gestört und der Blutfluß sinkt. Diese Situation ist aber lebensbedrohend, weil es - infolge des Herzmuskelflimmerns - sogar zu einem Stillstand der Blutförderung kommen kann, was den Tod zur Folge hat. Patienten, die von Herzflattern bzw. -flimmern bedroht sind, wird deshalb vorbeugend ein sogenannter Defibrillator implantiert (siehe dazu beispielsweise DE-OS 39 19 498).

Defibrillatoren haben die Aufgabe, abnorme Herzfrequenzen zu detektieren, d.h. kritische Situationen zu erkennen. Mittels großflächiger Elektroden, von denen wenigstens eine außen am Herzen appliziert wird, wird dem Myocard ein Stromimpuls aufgeprägt. Dieser Stromimpuls, der mono- oder biphasisch sein kann, bringt die Muskelzellen in den refraktären Zustand, so daß der Herzmuskel anschließend wieder normal arbeiten kann, d.h. er wird zu koordinierter Bewegung und normaler Leistung angeregt.

Der Strom- bzw. Reizimpuls dauert im allgemeinen 4 bis 10 ms und wirkt mit einer maximalen Stromdichte von 100 bis 200 mA/cm². Ein technisches Problem besteht nun darin, daß bei einer derart hohen Stromdichte, d.h. bei einer Amplitude des Stromimpulses von mehr als 10 A, auch bei einer kurzen Impulsdauer von 10 ms an den Elektroden Gas entwickelt wird und die Elektrodenpolarisation den Energiebedarf erhöht. Häufig müssen auch mehrere Impulse angewendet werden, und man beobachtet dann, daß der Innenwiderstand steigt. Eine Ursache dafür ist in der auf der Elektrode haftenden Gasschicht zu sehen.

Die Elektroden von Defibrillatoren, sogenannte Patches, werden aus einem dünnen Titannetz hergestellt und haben beispielsweise eine Fläche von 20 cm² (siehe dazu: "RBM - Revue européenne de technologie biomédicale", Vol. 12 (1990), No. 3, Seite 125: Nr. 464). Netze werden deshalb verwendet, damit den Anforderungen an die mechanische Beweglichkeit genügt werden kann; Titan andererseits ist ein körperverträgliches Material. Die Titannetze sind auf der Rückseite, d.h. einseitig, in eine Schicht aus Silicongummi eingebettet, durch die sie gestützt werden.

Von Nachteil ist dabei, daß bei monophasischen Impulsen an den anodisch belasteten Elektroden Oxidschichten gebildet werden und die elektrochemische Doppelschichtkapazität sinkt, beispielsweise, mit der Zahl der Impulse, sukzessiv von 20 $\mu F/cm^2$ auf 1 $\mu F/cm^2$; dadurch steigt die Polarisation beim Impuls. Als wesentlicher Nachteil der Titannetze ist aber die Gasentwicklung an den Elektroden während des Stromimpulses anzusehen und damit eine mangelnde Bioverträglichkeit.

Aufgabe der Erfindung ist es, Elektroden für Defibrillatoren, welche ein Netz aus Titan aufweisen, derart auszugestalten, daß sie eine gleichbleibend hohe Doppelschichtkapazität aufweisen und an ihnen keine Gasentwicklung erfolgt.

Dies wird erfindungsgemäß dadurch erreicht, daß das Titannetz eine poröse Schicht aus einem Carbid, Nitrid oder Carbonitrid oder einem elektronisch leitenden Oxid wenigstens eines der Metalle aus der Gruppe der Elemente der vierten und fünften Nebengruppe des Periodensystems und Wolfram aufweist.

Die Metalle der vierten und fünften Nebengruppe des Periodensystems der Elemente, die - ebenso wie Wolfram (aus der 6. Nebengruppe) - zu den Übergangsmetallen zählen, sind im einzelnen:
- 4. Nebengruppe: Titan, Zirkonium, Hafnium;
- 5. Nebengruppe: Vanadium, Niob, Tantal.

Carbide MeC und Nitride MeN der genannten Art (Me = Metall) sind beispielsweise TiC, TiN, ZrC, ZrN, TaC und TaN. Es handelt sich dabei im wesentlichen um die stöchiometrischen Verbindungen, Abweichungen vom stöchiometrischen Verhältnis können aber gegeben sein. Die Carbonitride weisen die Zusammensetzung $MeC_xN_{1-x}$ auf, wobei x einen Wert zwischen 0 und 1 annehmen kann; beispielhaft sei hierzu die Verbindung $TiC_{0,5}N_{0,5}$ genannt. In allen Fällen können auch "gemischte" Verbindungen vorliegen, d.h. Me kann für verschiedene Metalle stehen; derartige Verbindungen sind beispielsweise das Carbid (W,Ti)C und das Nitrid (Ti,Nb)N. Darüber hinaus können die genannten Verbindungen auch in Form von Gemischen zum Einsatz gelangen.

Die Carbide, Nitride und Carbonitride der genannten Metalle sind elektronisch gut leitend. Von den Metalloxiden kommen diejenigen Verbindungen in Betracht, die elektronisch leitend sind, beispielsweise Titanoxide wie $Ti_2O$.

Die porösen Schichten aus den genannten Metallverbindungen weisen vorzugsweise eine Dicke zwischen 5 und 30 $\mu m$ auf.

Die Elektroden nach der Erfindung werden vorteilhaft in der Weise hergestellt, daß ein dünnes Titannetz durch reaktives Ionenplattieren mittels einer Sputterquelle mit einem porösen Carbid, Nitrid, Carbonitrid oder Oxid wenigstens eines der folgenden Metalle beschichtet wird: Ti, Zr, Hf, V, Nb, Ta und W. Bei Ionenbeschußdichten $\leq 1$ mA/cm² werden dabei poröse Schichten mit günstiger Struktur erhalten, die eine hohe Doppelschichtkapazität gewährleistet. Um eine gute Anbindung an das Titannetz zu erreichen, können die Schichten auch aus zwei Bereichen aufgebaut sein, und zwar aus einer dichten Grundschicht, die bei hoher Ionenbeschußdichte, d.h. $\geq 2$ mA/cm$^2$, hergestellt wird, und die - durch sukzessives Absenken der Ionenstromdichte - fließend in die poröse Schicht übergeht.

Anhand von Ausführungsbeispielen soll die Erfindung noch näher erläutert werden.

Für die Untersuchungen wurden in einer Meßzelle jeweils zwei Netze (Fläche: ca. 1,7 cm$^2$) symmetrisch im Abstand von 2 cm angeordnet, so daß eine gleichmäßige Stromdichte gewährleistet war. Rückseitig wurden die Elektroden durch eine Scheibe aus Silicongummi gestützt, der elektrische Kontakt wurde seitlich herausgeführt. Als Elektrolyt diente 0,15 M NaCl mit Phosphatpuffer (pH 7). Die Messungen, die sämtlich bei Raumtemperatur durchgeführt wurden, erfolgten an unbeschichteten Ti-Netzen bzw. an mit porösem TiN beschichteten Ti-Netzen (Schichtdicke: ca. 15 $\mu$m).

Vor der Belastung durch Stromimpulse wurde die Impedanz der symmetrisch angeordneten Ti-Netze gemessen. Bei unbeschichteten Netzen ergibt sich bei einer Frequenz von 1 Hz eine Kapazität von 22,5 $\mu$F/cm$^2$; die Kapazität sinkt mit steigender Frequenz. Bezogen auf die wahre Oberfläche der Netze, d.h. auf die Oberfläche der Drähte, die sich - bei einem Drahtdurchmesser von ca. 100 $\mu$m und einer Maschenweite von ca. 500 $\mu$m - zu 1,25 cm$^2$ pro Quadratzentimeter ergibt, beträgt die Kapazität 18 $\mu$F/cm$^2$; dieser Wert wird auch an glatten Ti-Blechen gefunden. Im Vergleich dazu weisen mit porösem TiN beschichtete Ti-Netze bei 1 Hz eine um den Faktor 535 höhere Kapazität auf, bei 1 kHz um den Faktor 100. Diese hohe Doppelschichtkapazität ist auf die poröse Struktur der TiN-Schicht zurückzuführen, deren Oberfläche ca. 90 cm$^2$ pro Quadratzentimeter beträgt.

Bei den unbeschichteten Ti-Netzen ändert sich die Spannung während eines galvanostatischen biphasischen Impulses (Stromdichte: 10 mA/cm$^2$) zunächst linear und mündet dann in ein Plateau ein, wobei eine Gasentwicklung erfolgt. Anschließend wirkt der zweite Teil des biphasischen Impulses und die Stromrichtung wechselt; das Potential durchwandert dabei ein Intervall von 4 V. Am Ende des Impulses ergibt sich eine Polarisation von 1,7 V, die langsam abgebaut wird. Wird die Stromdichte von 10 mA/cm$^2$ auf 100 mA/cm$^2$ erhöht, so beträgt die Polarisation am Ende des Impulses 3,7 V, d.h. sie verdoppelt sich nur, während die Stromdichte verzehnfacht wurde. Ursache dafür sind die Faraday'schen Reaktionen, d.h. Oxidation und Gasentwicklung.

Anschließend wurden die unbeschichteten Ti-Netze mit 100 biphasischen Impulsen (100 mA/cm$^2$, 10/10 ms) belastet. Dabei zeigt sich, daß die Kapazität mit der Zahl der Impulse abnimmt (bei 100 Hz beispielsweise von 26 $\mu$F/cm$^2$ auf 9 $\mu$F/cm$^2$); bei den ersten Impulsen ist dieser Effekt ausgeprägter als bei den nachfolgenden. Insgesamt ist die Abnahme (auf ein Drittel) bei biphasischen Impulsen aber geringer als bei monophasischen Impulsen und anodischem Strom (Abnahme auf ein Zehntel). Infolge der sinkenden Doppelschichtkapazität ergibt sich mit zunehmender Impulszahl eine Erhöhung der Polarisation.

Bei der Messung an einer symmetrischen Anordnung aus zwei mit porösem TiN beschichteten Ti-Netzen zeigt sich folgendes. Bei einer Belastung mit biphasischen galvanostatischen Impulsen mit einer Stromdichte von 10 mA/cm$^2$ ergibt sich eine maximale Spannung von 60 mV. Wird die Stromdichte auf 100 mA/cm$^2$ erhöht, so steigt die Spannung auf 360 mV. Dieser Spannungswert ändert sich auch dann nicht, wenn den Elektroden 100 Impulse mit einer Stromdichte von 100 mA/cm$^2$ aufgeprägt werden; auch die Kapazität bleibt stabil.

Die experimentellen Ergebnisse zeigen, daß unbeschichtete Ti-Netze sowohl bei mono- als auch bei biphasischen Impulsen an Doppelschichtkapazität verlieren; die Polarisation steigt, nähert sich allerdings mit der Zahl der Impulse einem Grenzwert. Beim biphasischen Impuls sind die Änderungen dabei weniger ausgeprägt als beim monophasischen anodischen Impuls. In allen Fällen kommt es an den Elektroden zu einer Gasentwicklung. Werden dagegen mit porösem TiN beschichtete Ti-Netze verwendet, so wird die Polarisation reduziert und das elektrochemische Verhalten bleibt stabil. Dadurch unterbleibt eine Gasentwicklung, so daß die Bioverträglichkeit verbessert wird. Derartige Elektroden sind somit hervorragend als Patches für implantierbare Defibrillatoren geeignet.

**Patentansprüche**

1. Elektroden für implantierbare Defibrillatoren in Form eines dünnen Titannetzes, **dadurch gekennzeichnet,** daß das Titannetz eine poröse Schicht aus einem Carbid, Nitrid oder Carbonitrid oder einem elektronisch leitenden Oxid wenigstens eines der Metalle aus der Gruppe der Elemente der vierten und fünften Nebengruppe des Periodensystems und Wolfram aufweist.

2. Elektrode nach Anspruch 1, **dadurch gekennzeichnet,** daß die poröse Schicht eine Dicke zwischen 5 und 30 $\mu$m besitzt.

3. Verfahren zur Herstellung einer Elektrode nach Anspruch 1 oder 2, **dadurch gekennzeichnet,** daß ein dünnes Titannetz durch reaktives Ionenplattieren mittels einer Sputterquelle mit einem porösen Carbid, Nitrid, Carbonitrid oder Oxid wenigstens eines der Metalle Titan, Zirkonium, Hafnium, Vanadium, Niob, Tantal und Wolfram beschichtet wird.

4. Verfahren nach Anspruch 3, **dadurch gekennzeichnet,** daß die Beschichtung bei einer lonenstromdichte $\leq 1$ mA/cm$^2$ duchgeführt wird.

5. Verfahren nach Anspruch 3 oder 4, **dadurch gekennzeichnet,** daß während des Beschichtungsvorgangs die lonenstromdichte sukzessive von einem Wert $\geq 2$ mA/cm$^2$ auf einen Wert $\leq 1$ mA/cm$^2$ abgesenkt wird.

Europäisches
Patentamt

**EUROPÄISCHER RECHERCHENBERICHT**

Nummer der Anmeldung

EP    91 11 2258

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | EP-A-0 115 778 (SIEMENS) <br> * das ganze Dokument * <br> --- | 1-3 | A61N1/05 |
| A | EP-A-0 280 564 (INTERMEDICS) <br> * Spalte 5, Zeile 64 - Spalte 6, Zeile 8 * <br> ----- | 1 | |

**RECHERCHIERTE SACHGEBIETE (Int. Cl.5)**

A61N

Der vorliegende Recherchenbericht wurde für alle Patentansprüche erstellt

| Recherchenort | Abschlußdatum der Recherche | Prüfer |
|---|---|---|
| DEN HAAG | 13 NOVEMBER 1991 | LEMERCIER D.L.L. |